# EUROPEAN PATENT APPLICATION

(11) **EP 3 957 606 A1**
(43) Date of publication of application: **23.02.2022**
(21) Application number: 20791465.6
(22) Date of filing: 06.04.2020
(51) Int. Cl.: C01G 23/04, A61K 8/29, C01B 33/12, C08G 77/02, C08G 77/38

(54) **SILICA-COATED PARTICLES AND METHOD FOR PRODUCING SAME**

(30) Priority: 15.04.2019 JP 2019076911
(71) Applicant: SHIN-ETSU CHEMICAL CO., LTD., Tokyo (JP)
(72) Inventor: INOKUCHI Yoshinori, Annaka-shi, Gunma 379-0224 (JP)
(74) Representative: Mewburn Ellis LLP
(86) International application number: PCT/JP2020/015465
(87) International publication number: WO 2020/213444

(57) **Abstract**

Provided are: silica-coated particles which have satisfactory dispersibility in resins and liquid materials, have functional groups capable of reacting with resins, and also has satisfactory slipperiness as well as water repellency; and a method for producing the silica-coated particles. Silica-coated particles, in which the surface of each of solid particles (A) is coated with silica (B), a part or the whole of a hydroxyl group on the surface of the silica (B) is reacted with at least one component (C) selected from an organoalkoxysilane and a hydrolysate thereof and an organosilazane, wherein the amount of the silica (B) is 0.5 to 100 parts by mass relative to 100 parts by mass of the solid particles (A).

## Description

### TECHNICAL FIELD

This invention relates to silica-coated particles in which solid particles are coated on their surfaces with silica, and a method for preparing the same.

### BACKGROUND ART

Inorganic powders including metal powders, titanium oxide, zinc oxide, mica, talc, and barium sulfate and organic resin powders are commonly used in resins, coating compositions and cosmetics. Sometimes, these particles are coated on their surfaces with silica for the purposes of improving non-agglomeration, fluidity, dispersibility, photocatalytic activity inhibition, alkali/acid resistance, feeling, light diffusion, and the like.

Known methods for coating surfaces of solid particles with silica include a method of mixing solid particles with silica particles in dry state on such a mixer as a ball mill or hybridizer, while applying impact forces (Patent Document 1: JP-A H06-32725), and a method of removing water from a liquid consisting of a water dispersion of solid particles and silica sol (Patent Document 2: JP-A H04-348143). Also known are a method of dispersing solid particles in a silicic acid solution and allowing silica to precipitate on surfaces of the solid particles (Patent Document 3: JP-A 2002-87817) and a method of dispersing solid particles in an organic solvent and hydrolyzing a tetraalkoxysilane in the dispersion to form silica on surfaces of the solid particles (Patent Document 4: JP-A H11-193354).

However, these silica-coated particles are insufficient in dispersibility in particulate resins and liquids. Owing to silanol groups on silica surface, the silica-coated particles are sometimes more agglomerative or cohesive than non-silica-coated particles. Since the silica-coated particles have poor adhesion to resins, a problem arises that when a resin is loaded with the silica-coated particles and then cut, some particles spall off from the cut section.

When applied to cosmetics, the silica-coated particles are insufficient in such feeling as slipperiness. Although the silica-coated particles are hydrophilic, some cosmetics require water repellency.

### PRIOR ART DOCUMENTS

### PATENT DOCUMENTS

Patent Document 1: JP-A H06-32725
Patent Document 2: JP-A H04-348143
Patent Document 3: JP-A 2002-87817
Patent Document 4: JP-A HI 1-193354

### SUMMARY OF INVENTION

### TECHNICAL PROBLEM

An object of the invention, which has been made under the above-mentioned circumstances, is to provide silica-coated particles which are effectively dispersible in resins and liquids, have a functional group capable of reacting with resins, or have good slipperiness or water repellency, and a method for preparing the same.

### SOLUTION TO PROBLEM

Making extensive investigations to attain the above object, the inventor has found that the outstanding problem associated with silica coating can be solved by reacting some or all of hydroxy groups on silica surfaces with (C) at least one compound selected from an organoalkoxysilane, hydrolyzate thereof, and organosilazane. The invention is predicated on this finding.

Accordingly, the invention provides silica-coated particles and a method for preparing the same, as defined below.
[1] Silica-coated particles comprising (A) solid particles each of which is surface-coated with (B) silica, wherein some or all hydroxy groups on the surface of the silica (B) have reacted with (C) at least one compound selected from an organoalkoxysilane, hydrolyzate thereof, and organosilazane, and 0.5 to 100 parts by weight of the silica (B) is present per 100 parts by weight of the solid particles (A).
[2] The silica-coated particles of [1] wherein the silica (B) is a hydrolytic condensate of (D) a tetraalkoxysilane.
[3] The silica-coated particles of [1] or [2] wherein the solid particles (A) are inorganic particles.
[4] The silica-coated particles of [1] or [2] wherein the solid particles (A) are organic particles.
[5] The silica-coated particles of any one of [1] to [4] wherein the organoalkoxysilane as component (C) is selected from an organotrialkoxysilane of the formula: R¹Si(OR²)₃, a diorganodialkoxysilane of the formula: R³₂Si(OR⁴)₂, and a trimethylalkoxysilane of the formula: (CH₃)₃SiOR⁵ wherein R¹ and R³ each are a C₁-C₂₀ monovalent organic group, R², R⁴ and R⁵ each are an unsubstituted C₁-C₆ monovalent hydrocarbon group, and the organosilazane is hexamethyldisilazane of the formula: (CH₃)₃SiNHSi(CH₃)₃.
[6] A method for preparing the silica-coated particles of any one of [1] to [5], comprising the steps of:
   (i) preparing a liquid containing (A) solid particles, (E) an alkaline substance, (F) at least one compound selected from a cationic surfactant and a cationic water-soluble polymer, and (G) water,
   (ii) adding (D) a tetraalkoxysilane to the liquid resulting from step (i), and subjecting the tetraalkoxysilane to hydrolysis and condensation to coat surfaces of the solid particles (A) with silica (B), thereby obtaining a dispersion of silica-coated particles,
   (iii) adding an extra of the alkaline substance (E) to the dispersion of silica-coated particles resulting from step (ii) and optionally, further adding (F) at least one compound selected from a cationic surfactant and a cationic water-soluble polymer thereto, thereby obtaining a dispersion having extra components added thereto, and
   (iv) adding (C) at least one compound selected from an organoalkoxysilane, hydrolyzate thereof, and organosilazane to the dispersion having extra components added thereto resulting from step (iii), and letting some or all hydroxy groups on the surfaces of the silica (B) react with (C) the at least one compound selected from an organoalkoxysilane, hydrolyzate thereof, and organosilazane.

### ADVANTAGEOUS EFFECTS OF INVENTION

According to the invention, silica-coated particles which are effectively dispersible in resins and liquids, have a functional group capable of reacting with resins, or have good slipperiness or water repellency are provided as well as a method for preparing the same.

### DESCRIPTION OF EMBODIMENTS

Now the invention is described in detail.

### [Component (A)]

Component (A): solid particles serve as nuclei of the silica-coated particles of the invention. Solid particles of one type or in admixture of two or more types may be used. The solid particles may be either inorganic or organic particles, have any geometrical shape such as spherical, polyhedral, spindle, needle, plate or other shape, and be porous or non-porous. The volume average particle size (MV value) is preferably 0.01 to 100 µm, more preferably 0.1 to 50 µm. The volume average particle size is measured by a method which is selected in accordance with a particular particle size from microscope, light scattering, laser diffraction, liquid phase sedimentation, electric resistance, and other methods. For example, a particle size of 0.01 µm to less than 1 µm may be measured by the light scattering method, and a particle size of 1 µm to 100 µm may be measured by the electric resistance method.

Suitable inorganic particles include particles of iron oxide, nickel oxide, chromium oxide, zinc oxide, titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, aluminum silicate, magnesium silicate, aluminum magnesium silicate, calcium silicate, barium silicate, strontium silicate, metal tungstate salts, hydroxyapatite, vermiculite, higilite, bentonite, montmorillonite, hectolite, zeolite, ceramics, calcium secondary phosphate, alumina, aluminum hydroxide, boron nitride, boron nitride, and glass.

Suitable organic particles include particles of polyamide, polyacrylic acid-acrylate, polyester, polyethylene, polypropylene, polystyrene, styrene-acrylic acid copolymers, divinylbenzene-styrene copolymers, polyurethane, vinyl resins, urea resins, melamine resins, benzoguanamine, polymethylbenzoguanamine, tetrafluoroethylene, poly(methyl methacrylate), cellulose, silk, nylon, phenolic resins, epoxy resins, polycarbonate, polybutadiene rubber, acrylic rubber, urethane rubber, silicone rubber, and fluoro rubber.

### [Component (B)]

Component (B): silica is to cover surfaces of solid particles (A) as nuclei. Silica has a structure composed of SiO₂ units. Although the preparation of silica is not particularly limited, it is preferably obtained from hydrolytic condensation reaction of (D) a tetraalkoxysilane as will be described later. The tetraalkoxysilane (D) is represented by Si(OR⁶)₄ wherein R⁶ is an alkyl group. The alkyl group is preferably of 1 to 6 carbon atoms. Exemplary alkyl groups include methyl, ethyl, propyl, butyl, pentyl and hexyl, with methyl and ethyl being preferred in view of reactivity. Specifically, tetramethoxysilane and tetraethoxysilane are preferred, with tetramethoxysilane being more preferred.

### [Component (C)]

Component (C) is at least one compound selected from an organoalkoxysilane, hydrolyzate thereof, and organosilazane, which may be used alone or in admixture of two or more.

Although the organoalkoxysilane is not particularly limited, examples of the organoalkoxysilane include organotrialkoxysilanes of the formula: R¹Si(OR²)₃, diorganodialkoxysilanes of the formula: R³₂Si(OR⁴)₂, and trimethylalkoxysilanes of the formula: (CH₃)₃SiOR⁵ wherein R¹ and R³ each are a C₁-C₂₀ monovalent organic group, R², R⁴ and R⁵ each are an unsubstituted C₁-C₆ monovalent hydrocarbon group, which may be used alone or in admixture of two or more.

R¹ and R³ each are a C₁-C₂₀ monovalent organic group, preferably a C₁-C₁₀ unsubstituted or substituted monovalent organic group. Examples include alkyl groups such as methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, and eicosyl; cycloalkyl groups such as cyclobutyl, cyclopentyl, cyclohexyl and cycloheptyl; aryl groups such as phenyl, tolyl and naphthyl; aralkyl groups such as benzyl, phenethyl, and β-phenylpropyl; alkenyl groups such as vinyl and allyl; and substituted forms of the foregoing hydrocarbon groups in which some or all of the carbon-bonded hydrogen atoms are substituted by atoms, typically halogen atoms (e.g., fluorine, chlorine, bromine and iodine atoms), and/or substituent groups such as acryloyloxy, methacryloyloxy, epoxy, glycidoxy, amino, mercapto, carboxy, alkylene glycol, polyoxyalkylene, aminoalcohol, and phosphorylcholine.

R², R⁴ and R⁵ each are an unsubstituted C₁-C₆ monovalent hydrocarbon group. Examples include methyl, ethyl, propyl, butyl, pentyl and hexyl, with methyl being preferred in view of reactivity.

Examples of the hydrolyzate include R¹Si(OH)₃, R³₂Si(OH)₂, and (CH₃)₃SiOH.

Although the organosilazane is not particularly limited, hexamethyldisilazane of the formula: (CH₃)₃SiNHSi(CH₃)₃ is preferred.

### [Silica-coated particles]

The silica-coated particles of the invention are defined as comprising (A) solid particles serving as nuclei, which are coated on their surfaces with (B) silica, wherein some or all hydroxy groups on the surface of the silica (B) have reacted with (C) at least one compound selected from an organoalkoxysilane, hydrolyzate thereof, and organosilazane.

The amount of the silica (B) is 0.5 to 100 parts by weight, preferably 1 to 50 parts by weight, more preferably 3 to 20 parts by weight per 100 parts by weight of the solid particles (A). If the amount of silica (B) is less than 0.5 part by weight, neither improvements in non-agglomeration, fluidity, dispersibility, photocatalytic activity inhibition, alkali and acid resistance, and feeling nor properties such as light diffusion are developed. If the amount of silica (B) exceeds 100 parts by weight, it is difficult for solid particles (A) to exert their properties.

The covering silica may take a film or particulate form. With respect to the coating state, silica may cover portions or the entirety of the surface of solid particle (A), preferably cover the overall surface of solid particle (A) substantially without leaving gaps. The coating state can be observed, for example, under an electron microscope.

The silica-coated particles may have any geometrical shape such as spherical, polyhedral, spindle, needle, plate or other shape, and be porous or non-porous. The volume average particle size is preferably 0.01 to 100 µm, more preferably 0.1 to 50 µm.

The silica-coated particles of the invention are such that some or all hydroxy groups on the surface of silica (B) have reacted with (C) at least one compound selected from an organoalkoxysilane, hydrolyzate thereof, and organosilazane. It is acceptable that not all hydroxy groups on the surface of silica (B) have reacted with component (C), that is, some hydroxy groups remain.

The amount of component (C) may be sufficient to react with hydroxy groups on the surface of covering silica (B). Although the necessary amount of component (C) varies depending on the specific surface area of particles, properties such as dispersibility and water repellency, and the desired degree of reactive functional groups, the amount is preferably 0.01 to 20 parts by weight, more preferably 0.05 to 10 parts by weight per 100 parts by weight of solid particles (A) and silica (B) combined.

### [Method for preparing silica-coated particles]

The method for preparing the silica-coated particles according to the invention is, for example, a method comprising the following steps of:
(i) preparing a liquid containing (A) solid particles, (E) an alkaline substance, (F) at least one compound selected from a cationic surfactant and a cationic water-soluble polymer, and (G) water,
(ii) adding (D) a tetraalkoxysilane to the liquid resulting from step (i), and subjecting the tetraalkoxysilane to hydrolysis and condensation to coat the surface of the solid particles (A) with silica (B), thereby obtaining a dispersion of silica-coated particles,
(iii) adding an extra of the alkaline substance (E) to the dispersion of silica-coated particles resulting from step (ii) and optionally, further adding (F) at least one compound selected from a cationic surfactant and a cationic water-soluble polymer thereto, thereby obtaining a dispersion having extra components added thereto, and
(iv) adding (C) at least one compound selected from an organoalkoxysilane, hydrolyzate thereof, and organosilazane to the dispersion having extra components added thereto resulting from step (iii), and letting some or all hydroxy groups on the surface of the silica (B) react with (C) at least one compound selected from an organoalkoxysilane, hydrolyzate thereof, and organosilazane.

Step (i) is to prepare a liquid containing (A) solid particles, (E) an alkaline substance, (F) at least one compound selected from a cationic surfactant and a cationic water-soluble polymer, and (G) water. In step (i), the liquid containing components (A), (E), (F) and (G) is obtained by mixing the components. As to the solid particles (A), a water dispersion of component (A) which is separately prepared may be used. The alkaline substance (E) may be added after components (A), (F) and (G) are mixed.

### [Component (E)]

The alkaline substance (E) functions as a catalyst for the hydrolytic condensation reaction of the tetraalkoxysilane (D) in step (ii). The alkaline substance may be used alone or in admixture of two or more.

The alkaline substance used herein is not particularly limited. Examples thereof include alkali metal hydroxides such as potassium hydroxide, sodium hydroxide and lithium hydroxide; alkaline earth metal hydroxides such as calcium hydroxide and barium hydroxide; alkali metal carbonates such as potassium carbonate and sodium carbonate; ammonia; tetraalkylammonium hydroxides such as tetramethylammonium hydroxide and tetraethylammonium hydroxide; and amines such as monomethylamine, monoethylamine, monopropylamine, monobutylamine, monopentylamine, dimethylamine, diethylamine, trimethylamine, triethanolamine, and ethylenediamine. Inter alia, ammonia is preferred because it can be readily removed from the powder of silica-coated particles by volatilization. Any commercially available ammonia aqueous solution may be used as the ammonia.

The amount of component (E) added is preferably such that the liquid containing components (A), (E), (F) and (G) in step (i) may have a pH value in the range of 9.0 to 12.0, more preferably 9.5 to 11.0. When the alkaline substance is added in such an amount as to provide pH 9.0 to 12.0, the progress of hydrolytic condensation reaction of tetraalkoxysilane (D) and the coverage of surfaces of solid particles (A) with silica are more ensured.

### [Component (F)]

The compound (F) selected from a cationic surfactant and a cationic water-soluble polymer may be used alone or in suitable combination of two or more. Component (F) functions to promote the condensation reaction of tetraalkoxysilane hydrolyzate to form silica. Also, there is a possibility that component (F) has a function of helping the thus formed silica adsorb to the surfaces of solid particles (A). In some cases, component (F) can function as a dispersant for solid particles (A).

Examples of the cationic surfactant include alkyltrimethylammonium salts, dialkyldimethylammonium salts, polyoxyethylene alkyldimethylammonium salts, dipolyoxyethylene alkylmethylammonium salts, tripolyoxyethylene alkylammonium salts, alkylbenzyldimethylammonium salts, alkylpyridinium salts, monoalkylamine salts, and monoalkylamidoamine salts.

Examples of the cationic water-soluble polymer include dimethyldiallylammonium chloride polymers, vinylimidazoline polymers, methylvinylimidazolium chloride polymers, ethyl acrylate trimethylammonium chloride polymers, ethyl methacrylate trimethylammonium chloride polymers, (acrylamidopropyl)trimethylammonium chloride polymers, (methacrylamidopropyl)trimethylammonium chloride polymers, epichlorohydrin/dimethylamine polymers, ethyleneimine polymers, quaternized ethyleneimine polymers, allylamine hydrochloride polymers, polylysine, cationic starch, cationic cellulose, and chitosan, as well as derivatives of the foregoing having a monomer containing a nonionic or anionic group copolymerized therewith.

The amount of component (F) added is preferably 0.01 to 2 parts by weight, more preferably 0.02 to 1 part by weight per 100 parts by weight of water in the liquid containing components (A), (E), (F) and (G). If the amount of component (F) is less than 0.01 part by weight, there may be left some silica which has not covered surfaces of solid particles (A). If the amount of component (F) exceeds 2 parts by weight, there may be left some silica which has not covered surfaces of solid particles (A).

### [Component (G)]

Component (G) is water, which may be purified water or the like. The amount of water is preferably such that solid particles (A) account for 1 to 50% by weight, more preferably 5 to 30% by weight of the liquid containing components (A), (E), (F) and (G).

As the dispersant for solid particles (A), a nonionic surfactant, ampholytic surfactant or nonionic water-soluble polymer may be blended. The dispersant may be used alone or in admixture of two or more. Examples of the nonionic surfactant include polyoxyethylene alkyl ethers, polyoxyethylene polyoxypropylene alkyl ethers, polyoxyethylene alkyl phenyl ethers, polyethylene glycol fatty acid esters, sorbitan fatty acid esters, polyoxyethylene sorbitan fatty acid esters, polyoxyethylene sorbit fatty acid esters, glycerin fatty acid esters, polyoxyethylene glycerin fatty acid esters, polyglycerin fatty acid esters, propylene glycol fatty acid esters, polyoxyethylene castor oil, polyoxyethylene hydrogenated castor oil, polyoxyethylene hydrogenated castor oil fatty acid esters, polyoxyethylenealkylamines, polyoxyethylene fatty acid amides, polyoxyethylene-modified organopolysiloxanes, and polyoxyethylene polyoxypropylene-modified organopolysiloxanes. Examples of the ampholytic surfactant include alkyldimethylamine oxides, alkyldimethylcarboxybetaines, alkylamidopropyldimethylcarboxybetaines, alkylhydroxysulfobetaines, and alkylcarboxymethyl hydroxyethylimidazolinium betaines. Examples of the nonionic water-soluble polymer include guar gum, starch, xanthane gum, methylcellulose, ethylcellulose, hydroxypropyl methyl cellulose, hydroxyethyl cellulose, hydroxypropyl cellulose, polyvinyl alcohol, polyvinyl methyl ether, polyvinyl pyrrolidone, polyethylene glycol, polyoxyethylene/polyoxypropylene copolymers, polyethyl acrylate, and polyacrylamide.

Step (ii) is to add (D) a tetraalkoxysilane to the liquid resulting from step (i), and subjecting the tetraalkoxysilane to hydrolysis and condensation to coat surfaces of the solid particles (A) with silica (B), thereby obtaining a dispersion of silica-coated particles. The condensate of tetraalkoxysilane (D) is silica (B). The thus formed silica (B) attaches to surfaces of solid particles (A) for thereby coating surfaces of solid particles (A) with silica (B). Although the tetraalkoxysilane (D) is as defined above, a hydrolyzed tetraalkoxysilane in which some or all alkoxy groups have been hydrolyzed, or a partially condensed tetraalkoxysilane may also be used.

The amount of component (D) added is preferably such that the amount of silica (B) is 0.5 to 100 parts by weight, more preferably 1 to 50 parts by weight per 100 parts by weight of solid particles (A).

Component (D) is added with stirring by a conventional agitator such as a propeller impeller, flat puddle or anchor impeller. Component (D) may be added all at once, but preferably over a certain time. The addition time is preferably in the range of 1 minute to 6 hours, more preferably 10 minutes to 3 hours. The temperature is preferably 0 to 60°C, more preferably 0 to 40°C. A temperature in the range of 0 to 60°C ensures that surfaces of solid particles (A) are coated with silica (B). Even after the addition of component (D) is ended, stirring is continued until the completion of hydrolytic condensation reaction of component (D). For confirming that coated particles are obtained, a powder of such particles may be examined by observation of particle surfaces under electron microscope, elemental analysis of particle surfaces, or a hydrophilic test.

Step (iii) is to add an extra of the alkaline substance (E) to the dispersion of silica-coated particles resulting from step (ii) and optionally, further adding (F) at least one compound selected from a cationic surfactant and a cationic water-soluble polymer thereto, thereby obtaining a dispersion having extra components added thereto.

Component (E) functions as a catalyst for the reaction of hydroxy groups on silica surfaces with at least one compound selected from an organoalkoxysilane, hydrolyzate thereof, and organosilazane in step (iv). Since the amount of component (E) added in step (i) is insufficient to forward the hydrolytic condensation reaction, an extra amount is added in step (iii). If necessary, at least one compound (F) selected from a cationic surfactant and a cationic water-soluble polymer is additionally added.

The amount of component (E) added in step (iii) is preferably at least 5 times, more preferably 10 to 300 times the amount of component (E) added in step (i). When component (F) is added, the amount of component (F) added is preferably 0.01 to 2 parts by weight, more preferably 0.02 to 1 part by weight per 100 parts by weight of water in the liquid containing components (A), (E), (F) and (G).

Step (iv) is to add (C) at least one compound selected from an organoalkoxysilane, hydrolyzate thereof, and organosilazane to the dispersion having extra components added thereto resulting from step (iii), for thereby letting some or all hydroxy groups on the surfaces of the silica (B) react with (C) the at least one compound selected from an organoalkoxysilane, hydrolyzate thereof, and organosilazane. Component (C) is as defined above.

Although the amount of component (C) added is enough to react with hydroxy groups on the surfaces of the covering silica (B), the amount may be small enough to react with some hydroxy groups on the surfaces of the covering silica (B) as long as the desired properties are obtained.

Component (C) is added with stirring by a conventional agitator such as a propeller impeller, flat puddle or anchor impeller.

Component (C) may be added all at once although it may be added over a certain time. The temperature is preferably 0 to 60°C, more preferably 0 to 40°C. Even after the addition of component (C), stirring is continued until the completion of condensation reaction of component (C). To drive the condensation reaction to completion, stirring may be performed at an elevated temperature of about 40°C to about 100°C.

After the reaction of component (C), water is removed from the resulting water dispersion of the desired particles. Water removal can be performed by heating the water dispersion under atmospheric or reduced pressure. Exemplary are a technique of removing water from the dispersion under heating while keeping it static, a technique of removing water from the dispersion under heating while stirring and fluidizing it, a technique of spraying and dispersing the dispersion into a hot air stream through a spray dryer, and a technique of utilizing a fluidized heat medium. As a treatment prior to this operation, the dispersion may be concentrated by suitable means such as heat drying, separation by filtration, centrifugal separation or decantation, and if necessary, washed with water or alcohol.

If the product obtained by removing water from the water dispersion is in agglomerated state, it is disintegrated on a crusher such as a jet mill, ball mill or hammer mill.

Whether or not component (C) has undergone condensation reaction with hydroxy groups on surfaces of silica-coated solid particles is judged by a hydrophobic test when organic groups in component (C) are hydrophobic. For example, the coated particles are admitted into water or alcohol water, whereupon the coated particles are kept afloat thereon. When organic groups in component (C) are hydrophilic, the judgment is made by testing a change of hydrophilicity.

The silica-coated particles thus obtained may be used in resins, coating compositions, cosmetics, and the like. Because of the advantages of non-agglomeration and dispersion, they are suited for highly agglomerative, poorly dispersible particles.

### EXAMPLES

Examples and Comparative Examples are given below for further illustrating the invention althrough the invention is not limited thereto. It is noted that compositional "%" and "parts" are by weight unless otherwise stated.

### [Example 1]

A 2-L glass beaker was charged with 120 g of titanium oxide particles (trade name: Tipaque PFC-407 by Ishihara Sangyo Kaisha, Ltd.), 1.3 g of 40% dimethyldiallylammonium chloride polymer aqueous solution (trade name: ME Polymer H40W by Toho Chemical Industry Co., Ltd., average molecular weight 240,000), and 1,040 g of water, which were stirred at 6,000 rpm for 10 minutes using a homomixer. Then the charge was passed through a homogenizer under a pressure of 100 MPa, obtaining a water dispersion of titanium oxide particles.

The water dispersion of titanium oxide particles was analyzed using a particle size distribution analyzer based on dynamic light scattering principle (instrument N4Plus by Beckman Coulter), finding a volume average particle size of 340 nm.

The water dispersion of titanium oxide particles, 968 g, was transferred to a glass flask of 2 L volume equipped with a stirrer having an anchor impeller, to which 1.6 g of 2.9% ammonia water was added. At this point, the liquid was at pH 10.5. After the liquid was conditioned at a temperature of 5-10°C, 14.5 g (amount to provide 5.7 parts of silica after hydrolytic condensation reaction per 100 parts of titanium oxide particles) of tetramethoxysilane was added dropwise over 15 minutes. While the liquid temperature was kept at 5-10°C, stirring was continued for a further 1 hour, obtaining a water dispersion of silica-coated titanium oxide particles.

Next, 10 g of 29% ammonia water was added, and then 6.0 g of trimethylsilanol was added. While the liquid temperature was kept at 5-10°C, stirring was continued for 30 minutes. The liquid was heated at 70-75°C, after which stirring was continued at the temperature for 1 hour.

The resulting liquid was dewatered through a pressure filter. The dewatered product was transferred to a glass flask of 2 L volume equipped with a stirrer having an anchor impeller, to which 1,000 g of 50% methanol water was added, followed by stirring for 30 minutes and dewatering through a pressure filter. The dewatered product was transferred to a glass flask of 2 L volume equipped with a stirrer having an anchor impeller, to which 1,000 g of water was added, followed by stirring for 30 minutes and dewatering through a pressure filter, the same procedure was repeated once more. The dewatered product was dried in a hot air circulating dryer at a temperature of 105°C. The dry product was disintegrated on a jet mill, obtaining particles.

On analysis by a particle size distribution analyzer based on dynamic light scattering principle (instrument N4Plus by Beckman Coulter), the resulting particles had a volume average particle size of 350 nm.

The particles were admitted into water, followed by stirring. The particles were not dispersed in water, but kept afloat thereon. Further, the particles were admitted into 40 vol% methanol water, followed by stirring. The particles were not dispersed in methanol water, but kept afloat thereon. From these results, it was judged that trimethylsilanol had undergone condensation reaction with hydroxy groups on surfaces of silica-coated titanium oxide particles.

### [Example 2]

A water dispersion of titanium oxide particles was obtained as in Example 1. The water dispersion of titanium oxide particles, 968 g, was transferred to a glass flask of 2 L volume equipped with a stirrer having an anchor impeller, to which 1.6 g of 2.9% ammonia water was added. At this point, the liquid was at pH 10.5. After the liquid was conditioned at a temperature of 5-10°C, 14.5 g (amount to provide 5.7 parts of silica after hydrolytic condensation reaction per 100 parts of titanium oxide particles) of tetramethoxysilane was added dropwise over 15 minutes. While the liquid temperature was kept at 5-10°C, stirring was continued for a further 1 hour, obtaining a water dispersion of silica-coated titanium oxide particles.

Next, 10 g of 29% ammonia water was added, and then 5.4 g of hexamethyldisilazane was added. While the liquid temperature was kept at 5-10°C, stirring was continued for 30 minutes. The liquid was heated at 70-75°C, after which stirring was continued at the temperature for 1 hour.

The resulting liquid was dewatered through a pressure filter. The dewatered product was transferred to a glass flask of 2 L volume equipped with a stirrer having an anchor impeller, to which 1,000 g of 50% methanol water was added, followed by stirring for 30 minutes and dewatering through a pressure filter. The dewatered product was transferred to a glass flask of 2 L volume equipped with a stirrer having an anchor impeller, to which 1,000 g of water was added, followed by stirring for 30 minutes and dewatering through a pressure filter. The same procedure was repeated once more. The dewatered product was dried in a hot air circulating dryer at a temperature of 105°C. The dry product was disintegrated on a jet mill, obtaining particles.

On analysis by a particle size distribution analyzer based on dynamic light scattering principle (instrument N4Plus by Beckman Coulter), the resulting particles had a volume average particle size of 350 nm. The particles were admitted into water, followed by stirring. The particles were not dispersed in water, but kept afloat thereon. Further, the particles were admitted into 40 vol% methanol water, followed by stirring. The particles were not dispersed in methanol water, but kept afloat thereon. From these results, it was judged that hexamethyldisilazane had reacted with hydroxy groups on surfaces of silica-coated titanium oxide particles, i.e., was trimethylsilylated.

### [Comparative Example 1]

A water dispersion of titanium oxide particles was obtained as in Example 1. The water dispersion of titanium oxide particles, 968 g, was transferred to a glass flask of 2 L volume equipped with a stirrer having an anchor impeller, to which 1.6 g of 2.9% ammonia water was added. At this point, the liquid was at pH 10.5. After the liquid was conditioned at a temperature of 5-10°C, 14.5 g (amount to provide 5.7 parts of silica after hydrolytic condensation reaction per 100 parts of titanium oxide particles) of tetramethoxysilane was added dropwise over 15 minutes. While the liquid temperature was kept at 5-10°C, stirring was continued for a further 1 hour. The liquid was heated at 70-75°C, after which stirring was continued at the temperature for 1 hour, obtaining a water dispersion of silica-coated titanium oxide particles.

The resulting liquid was dewatered through a pressure filter. The dewatered product was transferred to a glass flask of 2 L volume equipped with a stirrer having an anchor impeller, to which 1,000 g of 50% methanol water was added, followed by stirring for 30 minutes and dewatering through a pressure filter. The dewatered product was transferred to a glass flask of 2 L volume equipped with a stirrer having an anchor impeller, to which 1,000 g of water was added, followed by stirring for 30 minutes and dewatering through a pressure filter. The same procedure was repeated once more. The dewatered product was dried in a hot air circulating dryer at a temperature of 105°C. The dry product was disintegrated on a jet mill, obtaining particles.

On analysis by a particle size distribution analyzer based on electric resistance principle (instrument Multisizer 3 by Beckman Coulter), the resulting particles had a volume average particle size of 3.5 µm. From these results, it was judged that the mass was disintegrated by a crusher into particles, but not to primary particles due to extremely strong cohesion. The resulting particles were admitted into water, followed by stirring. The particles were dispersible in water and hydrophilic.

### [Example 3]

A glass flask of 2 L volume equipped with a stirrer having an anchor impeller was charged with 150 g of polymethyl methacrylate spherical particles having a volume average particle size of 6 µm (trade name: Art Pearl J-7P by Negami Chemical Industry Co., Ltd.), 798 g of water, 1.5 g of 2.9% ammonia water, and 5 g of 30% lauryltrimethylammonium chloride aqueous solution (trade name: Cation BB by NOF Corp.). At this point, the liquid was at pH 10.1. After the liquid was conditioned at a temperature of 5-10°C, 24.3 g (amount to provide 6.4 parts of silica after hydrolytic condensation reaction per 100 parts of polymethyl methacrylate spherical particles) of tetramethoxysilane was added dropwise over 20 minutes. While the liquid temperature was kept at 5-10°C, stirring was continued for a further 1 hour, obtaining a water dispersion of silica-coated polymethyl methacrylate particles.

Next, 3 g of 30% cetyltrimethylammonium chloride aqueous solution (trade name: Kotamine 60W by Kao Corp.) and 10 g of 29% ammonia water were added to the dispersion, after which 2.1 g of phenyltrimethoxysilane was added. While the liquid temperature was kept at 5-10°C, stirring was continued for 30 minutes. The liquid was heated at 70-75°C, after which stirring was continued at the temperature for 1 hour.

The resulting liquid was dewatered through a pressure filter. The dewatered product was transferred to a glass flask of 2 L volume equipped with a stirrer having an anchor impeller, to which 1,000 g of 50% methanol water was added, followed by stirring for 30 minutes and dewatering through a pressure filter. The dewatered product was transferred to a glass flask of 2 L volume equipped with a stirrer having an anchor impeller, to which 1,000 g of water was added, followed by stirring for 30 minutes and dewatering through a pressure filter. The same procedure was repeated once more. The dewatered product was dried in a hot air circulating dryer at a temperature of 105°C. The dry product was disintegrated on a jet mill, obtaining particles.

On analysis by a particle size distribution analyzer based on electric resistance principle (instrument Multisizer 3 by Beckman Coulter), the resulting particles had a volume average particle size of 6 µm.

The particles were admitted into water, followed by stirring. The particles were not dispersed in water, but kept afloat thereon. Further, the particles were admitted into 40 vol% methanol water, followed by stirring. The particles were not dispersed in methanol water, but kept afloat thereon. From these results, it was judged that phenyltrimethoxysilane had undergone hydrolytic condensation reaction with hydroxy groups on surfaces of silica-coated polymethyl methacrylate particles.

### [Comparative Example 2]

A glass flask of 2 L volume equipped with a stirrer having an anchor impeller was charged with 150 g of polymethyl methacrylate spherical particles having a volume average particle size of 6 µm (trade name: Art Pearl J-7P by Negami Chemical Industry Co., Ltd.), 798 g of water, 1.5 g of 2.9% ammonia water, and 5 g of 30% lauryltrimethylammonium chloride aqueous solution (trade name: Cation BB by NOF Corp.). At this point, the liquid was at pH 10.1. After the liquid was conditioned at a temperature of 5-10°C, 24.3 g (amount to provide 6.4 parts of silica after hydrolytic condensation reaction per 100 parts of polymethyl methacrylate spherical particles) of tetramethoxysilane was added dropwise over 20 minutes. While the liquid temperature was kept at 5-10°C, stirring was continued for a further 1 hour. The liquid was heated at 70-75°C, after which stirring was continued at the temperature for 1 hour, obtaining a water dispersion of silica-coated polymethyl methacrylate particles.

The resulting liquid was dewatered through a pressure filter. The dewatered product was transferred to a glass flask of 2 L volume equipped with a stirrer having an anchor impeller, to which 1,000 g of 50% methanol water was added, followed by stirring for 30 minutes and dewatering through a pressure filter. The dewatered product was transferred to a glass flask of 2 L volume equipped with a stirrer having an anchor impeller, to which 1,000 g of water was added, followed by stirring for 30 minutes and dewatering through a pressure filter. The same procedure was repeated once more. The dewatered product was dried in a hot air circulating dryer at a temperature of 105°C. The dry product was disintegrated on a jet mill, obtaining particles.

On analysis by a particle size distribution analyzer based on electric resistance principle (instrument Multisizer 3 by Beckman Coulter), the resulting particles had a volume average particle size of 6 µm. The particles were admitted into water, followed by stirring. The particles were dispersible in water and hydrophilic.

### [Comparative Example 3]

A glass flask of 2 L volume equipped with a stirrer having an anchor impeller was charged with 150 g of polymethyl methacrylate spherical particles having a volume average particle size of 6 µm (trade name: Art Pearl J-7P by Negami Chemical Industry Co., Ltd.), 798 g of water, 1.5 g of 2.9% ammonia water, and 5 g of 30% lauryltrimethylammonium chloride aqueous solution (trade name: Cation BB by NOF Corp.). At this point, the liquid was at pH 10.1. After the liquid was conditioned at a temperature of 5-10°C, 24.3 g (amount to provide 6.4 parts of silica after hydrolytic condensation reaction per 100 parts of polymethyl methacrylate spherical particles) of tetramethoxysilane was added dropwise over 20 minutes. While the liquid temperature was kept at 5-10°C, stirring was continued for a further 1 hour, obtaining a water dispersion of silica-coated polymethyl methacrylate particles.

Next, 3 g of 30% cetyltrimethylammonium chloride aqueous solution (trade name: Kotamine 60W by Kao Corp.) was added to the dispersion, after which 2.1 g of phenyltrimethoxysilane was added. In the step of adding phenyltrimethoxysilane, no extra ammonia water was added. While the liquid temperature was kept at 5-10°C, stirring was continued for 30 minutes. The liquid was heated at 70-75°C, after which stirring was continued at the temperature for 1 hour.

The resulting liquid was dewatered through a pressure filter. The dewatered product was transferred to a glass flask of 2 L volume equipped with a stirrer having an anchor impeller, to which 1,000 g of 50% methanol water was added, followed by stirring for 30 minutes and dewatering through a pressure filter. The dewatered product was transferred to a glass flask of 2 L volume equipped with a stirrer having an anchor impeller, to which 1,000 g of water was added, followed by stirring for 30 minutes and dewatering through a pressure filter. The same procedure was repeated once more. The dewatered product was dried in a hot air circulating dryer at a temperature of 105°C. The dry product was disintegrated on a jet mill, obtaining particles.

On analysis by a particle size distribution analyzer based on electric resistance principle (instrument Multisizer 3 by Beckman Coulter), the resulting particles had a volume average particle size of 6 µm.

The particles were admitted into water, followed by stirring. The particles were dispersible in water and hydrophilic. From these results, it was judged that phenyltrimethoxysilane had not undergone hydrolytic condensation reaction with hydroxy groups on surfaces of silica-coated polymethyl methacrylate particles.

### [Example 4]

A glass beaker of 1 L volume was charged with 139 g of methylvinylpolysiloxane with a kinematic viscosity of 8.4 mm²/s, represented by the average formula (1) below, and 61 g of methylhydrogenpolysiloxane with a kinematic viscosity of 12 mm²/s, represented by the average formula (2) below, which were stirred for dissolution by operating a homomixer at 2,000 rpm. Next, 0.8 g of polyoxyethylene lauryl ether (molar number of ethylene oxide added = 9 moles) and 50 g of water were added. The mixture was stirred by a homomixer at 6,000 rpm whereupon it turned to an oil-in-water emulsion and showed a viscosity buildup. Stirring was continued for a further 15 minutes. With stirring at 2,000 rpm, 747 g of water was added to the emulsion, obtaining a white emulsion. The emulsion was passed through a homogenizer under a pressure of 40 MPa, whereby emulsion particles were more finely divided. The resulting emulsion was transferred to a glass flask of 1 L volume equipped with a stirrer having an anchor impeller. The emulsion was conditioned at a temperature of 15-20°C, after which, with stirring, a mixture of 0.5 g of an isododecane solution of platinum/vinyl-containing disiloxane complex (platinum content 0.5%) and 1 g of polyoxyethylene lauryl ether (molar number of ethylene oxide added = 9 moles) was added. Stirring was continued at the temperature for 12 hours, obtaining a water dispersion of silicone rubber particles. It is noted that in the formulae below, the bonding order of siloxane units is not limited to the illustrated one.

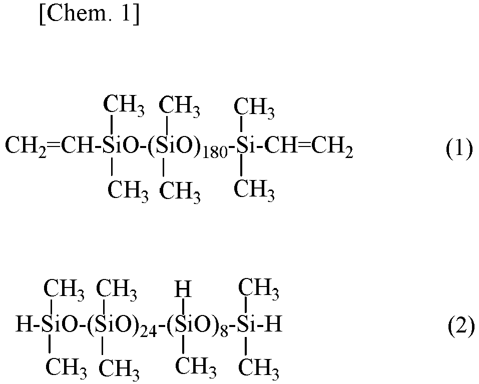

On observation under an optical microscope, the silicone rubber particles in the water dispersion had a spherical shape. On analysis by a particle size distribution analyzer based on electric resistance principle (instrument Multisizer 3 by Beckman Coulter), the particles had a volume average particle size of 2 µm.

Next, 750 g of the water dispersion of silicone rubber particles was transferred to a glass flask of 2 L volume equipped with a stirrer having an anchor impeller, to which 200 g of water, 1.5 g of 2.9% ammonia water, and 5 g of 30% lauryltrimethylammonium chloride aqueous solution (trade name: Cation BB by NOF Corp.) were added. At this point, the liquid was at pH 10.2. After the liquid was conditioned at a temperature of 5-10°C, 29.1 g (amount to provide 7.7 parts of silica after hydrolytic condensation reaction per 100 parts of silicone rubber particles) of tetramethoxysilane was added dropwise over 25 minutes. While the liquid temperature was kept at 5-10°C, stirring was continued for a further 1 hour, obtaining a water dispersion of silica-coated silicone rubber particles.

Next, 3 g of 30% cetyltrimethylammonium chloride aqueous solution (trade name: Kotamine 60W by Kao Corp.) and 10 g of 29% ammonia water were added to the dispersion, after which 1.8 g of trimethylmethoxysilane was added. While the liquid temperature was kept at 5-10°C, stirring was continued for 30 minutes. The liquid was heated at 70-75°C, after which stirring was continued at the temperature for 1 hour.

The resulting liquid was dewatered through a pressure filter. The dewatered product was transferred to a glass flask of 2 L volume equipped with a stirrer having an anchor impeller, to which 1,000 g of 50% methanol water was added, followed by stirring for 30 minutes and dewatering through a pressure filter. The dewatered product was transferred to a glass flask of 2 L volume equipped with a stirrer having an anchor impeller, to which 1,000 g of water was added, followed by stirring for 30 minutes and dewatering through a pressure filter. The same procedure was repeated once more. The dewatered product was dried in a hot air circulating dryer at a temperature of 80°C. The dry product was disintegrated on a jet mill, obtaining particles.

On analysis by a particle size distribution analyzer based on electric resistance principle (instrument Multisizer 3 by Beckman Coulter), the resulting particles had a volume average particle size of 2 µm.

The particles were admitted into water, followed by stirring. The particles were not dispersed in water, but kept afloat thereon. Further, the particles were admitted into 35 vol% methanol water, followed by stirring. The particles were not dispersed in methanol water, but kept afloat thereon. From these results, it was judged that trimethylmethoxysilane had undergone hydrolytic condensation reaction with hydroxy groups on surfaces of silica-coated silicone rubber particles.

### [Example 5]

A water dispersion of silicone rubber particles was obtained as in Example 4. The water dispersion of silicone rubber particles, 750 g, was transferred to a glass flask of 2 L volume equipped with a stirrer having an anchor impeller, to which 199 g of water, 1.5 g of 2.9% ammonia water, and 5 g of 30% lauryltrimethylammonium chloride aqueous solution (trade name: Cation BB by NOF Corp.) were added. At this point, the liquid was at pH 10.2. After the liquid was conditioned at a temperature of 5-10°C, 29.1 g (amount to provide 7.7 parts of silica after hydrolytic condensation reaction per 100 parts of silicone rubber particles) of tetramethoxysilane was added dropwise over 25 minutes. While the liquid temperature was kept at 5-10°C, stirring was continued for a further 1 hour, obtaining a water dispersion of silica-coated silicone rubber particles.

Next, 3 g of 30% cetyltrimethylammonium chloride aqueous solution (trade name: Kotamine 60W by Kao Corp.) and 10 g of 29% ammonia water were added to the dispersion, after which 2.1 g of phenyltrimethoxysilane was added. While the liquid temperature was kept at 5-10°C, stirring was continued for 30 minutes. The liquid was heated at 70-75°C, after which stirring was continued at the temperature for 1 hour.

The resulting liquid was dewatered through a pressure filter. The dewatered product was transferred to a glass flask of 2 L volume equipped with a stirrer having an anchor impeller, to which 1,000 g of 50% methanol water was added, followed by stirring for 30 minutes and dewatering through a pressure filter. The dewatered product was transferred to a glass flask of 2 L volume equipped with a stirrer having an anchor impeller, to which 1,000 g of water was added, followed by stirring for 30 minutes and dewatering through a pressure filter. The same procedure was repeated once more. The dewatered product was dried in a hot air circulating dryer at a temperature of 80°C. The dry product was disintegrated on a jet mill, obtaining particles.

On analysis by a particle size distribution analyzer based on electric resistance principle (instrument Multisizer 3 by Beckman Coulter), the resulting particles had a volume average particle size of 2 µm.

The particles were admitted into water, followed by stirring. The particles were not dispersed in water, but kept afloat thereon. Further, the particles were admitted into 10 vol% methanol water, followed by stirring. The particles were not dispersed in methanol water, but kept afloat thereon. From these results, it was judged that phenyltrimethoxysilane had undergone hydrolytic condensation reaction with hydroxy groups on surfaces of silica-coated silicone rubber particles.

### [Example 6]

A water dispersion of silicone rubber particles was obtained as in Example 4. The water dispersion of silicone rubber particles, 750 g, was transferred to a glass flask of 2 L volume equipped with a stirrer having an anchor impeller, to which 199 g of water, 1.5 g of 2.9% ammonia water, and 5 g of 30% lauryltrimethylammonium chloride aqueous solution (trade name: Cation BB by NOF Corp.) were added. At this point, the liquid was at pH 10.2. After the liquid was conditioned at a temperature of 5-10°C, 29.1 g (amount to provide 7.7 parts of silica after hydrolytic condensation reaction per 100 parts of silicone rubber particles) of tetramethoxysilane was added dropwise over 25 minutes. While the liquid temperature was kept at 5-10°C, stirring was continued for a further 1 hour, obtaining a water dispersion of silica-coated silicone rubber particles.

Next, 3 g of 30% cetyltrimethylammonium chloride aqueous solution (trade name: Kotamine 60W by Kao Corp.) and 10 g of 29% ammonia water were added to the dispersion, after which 1.9 g of 3-methacryloxypropyltrimethoxysilane was added. While the liquid temperature was kept at 5-10°C, stirring was continued for 30 minutes. The liquid was heated at 70-75°C, after which stirring was continued at the temperature for 1 hour.

The resulting liquid was dewatered through a pressure filter. The dewatered product was transferred to a glass flask of 2 L volume equipped with a stirrer having an anchor impeller, to which 1,000 g of 50% methanol water was added, followed by stirring for 30 minutes and dewatering through a pressure filter. The dewatered product was transferred to a glass flask of 2 L volume equipped with a stirrer having an anchor impeller, to which 1,000 g of water was added, followed by stirring for 30 minutes and dewatering through a pressure filter. The same procedure was repeated once more. The dewatered product was dried in a hot air circulating dryer at a temperature of 80°C. The dry product was disintegrated on a jet mill, obtaining particles.

On analysis by a particle size distribution analyzer based on electric resistance principle (instrument Multisizer 3 by Beckman Coulter), the resulting particles had a volume average particle size of 2 µm.

The particles were admitted into water, followed by stirring. The particles were not dispersed in water, but kept afloat thereon. Further, the particles were admitted into 10 vol% methanol water, followed by stirring. The particles were not dispersed in methanol water, but kept afloat thereon. From these results, it was judged that 3-methacryloxypropyltrimethoxysilane had undergone hydrolytic condensation reaction with hydroxy groups on surfaces of silica-coated silicone rubber particles.

### [Comparative Example 4]

A water dispersion of silicone rubber particles was obtained as in Example 4. The water dispersion of silicone rubber particles, 750 g, was transferred to a glass flask of 2 L volume equipped with a stirrer having an anchor impeller, to which 200 g of water, 1.5 g of 2.9% ammonia water, and 5 g of 30% lauryltrimethylammonium chloride aqueous solution (trade name: Cation BB by NOF Corp.) were added. At this point, the liquid was at pH 10.2. After the liquid was conditioned at a temperature of 5-10°C, 29.1 g (amount to provide 7.7 parts of silica after hydrolytic condensation reaction per 100 parts of silicone rubber particles) of tetramethoxysilane was added dropwise over 25 minutes. While the liquid temperature was kept at 5-10°C, stirring was continued for a further 1 hour. The liquid was heated at 70-75°C, after which stirring was continued at the temperature for 1 hour, obtaining a water dispersion of silica-coated silicone rubber particles.

The resulting liquid was dewatered through a pressure filter. The dewatered product was transferred to a glass flask of 2 L volume equipped with a stirrer having an anchor impeller, to which 1,000 g of 50% methanol water was added, followed by stirring for 30 minutes and dewatering through a pressure filter. The dewatered product was transferred to a glass flask of 2 L volume equipped with a stirrer having an anchor impeller, to which 1,000 g of water was added, followed by stirring for 30 minutes and dewatering through a pressure filter. The same procedure was repeated once more. The dewatered product was dried in a hot air circulating dryer at a temperature of 80°C. The dry product was disintegrated on a jet mill, obtaining particles.

On analysis by a particle size distribution analyzer based on electric resistance principle (instrument Multisizer 3 by Beckman Coulter), the resulting particles had a volume average particle size of 2 µm.

The particles were admitted into water, followed by stirring. The particles were dispersible in water and hydrophilic.

### [Comparative Example 5]

A water dispersion of silicone rubber particles was obtained as in Example 4. The water dispersion of silicone rubber particles, 750 g, was transferred to a glass flask of 2 L volume equipped with a stirrer having an anchor impeller, to which 200 g of water, 1.5 g of 2.9% ammonia water, and 5 g of 30% lauryltrimethylammonium chloride aqueous solution (trade name: Cation BB by NOF Corp.) were added. At this point, the liquid was at pH 10.2. After the liquid was conditioned at a temperature of 5-10°C, 29.1 g (amount to provide 7.7 parts of silica after hydrolytic condensation reaction per 100 parts of silicone rubber particles) of tetramethoxysilane was added dropwise over 25 minutes. While the liquid temperature was kept at 5-10°C, stirring was continued for a further 1 hour, obtaining a water dispersion of silica-coated silicone rubber particles.

Next, 3 g of 30% cetyltrimethylammonium chloride aqueous solution (trade name: Kotamine 60W by Kao Corp.) was added to the dispersion, after which 1.8 g of trimethylmethoxysilane was added. In the step of adding trimethylmethoxysilane, no extra ammonia water was added. While the liquid temperature was kept at 5-10°C, stirring was continued for 30 minutes. The liquid was heated at 70-75°C, after which stirring was continued at the temperature for 1 hour.

The resulting liquid was dewatered through a pressure filter. The dewatered product was transferred to a glass flask of 2 L volume equipped with a stirrer having an anchor impeller, to which 1,000 g of 50% methanol water was added, followed by stirring for 30 minutes and dewatering through a pressure filter. The dewatered product was transferred to a glass flask of 2 L volume equipped with a stirrer having an anchor impeller, to which 1,000 g of water was added, followed by stirring for 30 minutes and dewatering through a pressure filter. The same procedure was repeated once more. The dewatered product was dried in a hot air circulating dryer at a temperature of 80°C. The dry product was disintegrated on a jet mill, obtaining particles.

On analysis by a particle size distribution analyzer based on electric resistance principle (instrument Multisizer 3 by Beckman Coulter), the resulting particles had a volume average particle size of 2 µm.

The particles were admitted into water, followed by stirring. The particles were dispersible in water and hydrophilic. From these results, it was judged that trimethylmethoxysilane had not undergone hydrolytic condensation reaction with hydroxy groups on surfaces of silica-coated silicone rubber particles.

### [Viscosity of loaded liquid epoxy resin]

In a glass bottle, 70 g of liquid epoxy resin (trade name: ZX-1059 by Nippon Steel Chemical and Material Co., Ltd.) and 8 g of silica-coated silicone rubber particles in Table 1 were weighed and stirred with a spatula, whereby the silica-coated silicone rubber particles were uniformly dispersed in the resin. The dispersion was measured for viscosity at 25°C by a rotational viscometer, with the results shown in Table 1.

**[Table 1]**

| Silica-coated silicone rubber particles | Viscosity (mPa·s) |
|---|---|
| Non-loaded resin | 2,020 |
| Silica-coated silicone rubber particles of Comparative Example 3 | 5,450 |
| Silica-coated silicone rubber particles of Example 5 | 3,900 |

With respect to the viscosity of liquid epoxy resin loaded with silica-coated silicone rubber particles, the resin loaded with the particles of Example 5 in which hydroxy groups on silica surfaces have reacted with phenyltrimethoxysilane shows a lower viscosity than the resin loaded with the particles of Comparative Example 3 in which silica surfaces are not treated. It is thus judged that the particles of Example 5 in which hydroxy groups on silica surfaces have reacted with phenyltrimethoxysilane are more dispersible in and wettable to epoxy resins.

## Claims

1. Silica-coated particles comprising (A) solid particles each of which is surface-coated with (B) silica, wherein some or all hydroxy groups on the surface of the silica (B) have reacted with (C) at least one compound selected from an organoalkoxysilane, hydrolyzate thereof, and organosilazane, and 0.5 to 100 parts by weight of the silica (B) is present per 100 parts by weight of the solid particles (A).

2. The silica-coated particles of claim 1 wherein the silica (B) is a hydrolytic condensate of (D) a tetraalkoxysilane.

3. The silica-coated particles of claim 1 or 2 wherein the solid particles (A) are inorganic particles.

4. The silica-coated particles of claim 1 or 2 wherein the solid particles (A) are organic particles.

5. The silica-coated particles of any one of claims 1 to 4 wherein the organoalkoxysilane as component (C) is selected from an organotrialkoxysilane of the formula: R¹Si(OR²)₃, a diorganodialkoxysilane of the formula: R³₂Si(OR⁴)₂, and a trimethylalkoxysilane of the formula: (CH₃)₃SiOR⁵ wherein R¹ and R³ each are a C₁-C₂₀ monovalent organic group, R², R⁴ and R⁵ each are an unsubstituted C₁-C₆ monovalent hydrocarbon group, and the organosilazane is hexamethyldisilazane of the formula: (CH₃)₃SiNHSi(CH₃)₃.

6. A method for preparing the silica-coated particles of any one of claims 1 to 5, comprising the steps of:
(i) preparing a liquid containing (A) solid particles, (E) an alkaline substance, (F) at least one compound selected from a cationic surfactant and a cationic water-soluble polymer, and (G) water,
(ii) adding (D) a tetraalkoxysilane to the liquid resulting from step (i), and subjecting the tetraalkoxysilane to hydrolysis and condensation to coat surfaces of the solid particles (A) with silica (B), thereby obtaining a dispersion of silica-coated particles,
(iii) adding an extra of the alkaline substance (E) to the dispersion of silica-coated particles resulting from step (ii) and optionally, further adding (F) at least one compound selected from a cationic surfactant and a cationic water-soluble polymer thereto, thereby obtaining a dispersion having extra components added thereto, and
(iv) adding (C) at least one compound selected from an organoalkoxysilane, hydrolyzate thereof, and organosilazane to the dispersion having extra components added thereto resulting from step (iii), and letting some or all hydroxy groups on the surfaces of the silica (B) react with (C) the at least one compound selected from an organoalkoxysilane, hydrolyzate thereof, and organosilazane.
